# EUROPEAN PATENT APPLICATION

(11) **EP 2 737 846 A1**
(43) Date of publication of application: **04.06.2014**
(21) Application number: 13194921.6
(22) Date of filing: 28.11.2013
(51) Int. Cl.: A61B 5/00, A61B 5/0408

(54) **Biological information detecting device**

(30) Priority: 28.11.2012 JP 2012260263; 25.09.2013 JP 2013198756
(71) Applicant: Seiko Instruments Inc., Chiba-shi, Chiba (JP)
(72) Inventor: Muhishima, Katsuya, Chiba-shi, Chiba (JP); Takakura, Akira, Chiba-shi, Chiba (JP); Kato, Teruo, Chiba-shi, Chiba (JP)
(74) Representative: Miller Sturt Kenyon

(57) **Abstract**

Provided is a biological information detecting device including: a device main body (4); a heart rate detector (3a and 3b) that is integrally provided with the device main body (4) and includes an electrode (7a and 7b); and a fixing band (80) for mounting the device main body (4) and the heart rate detectors (3a and 3b) on a user, the fixing band (80) being formed of an extendable band-shaped member, in which the fixing band (80) includes adjusting portions (30) that are able to adjust an effective length of the fixing band (80), and the adjusting portions (30) include a first adjusting portion (50) and a second adjusting portion (60) that roughly and finely adjust the effective length of the fixing band (80), respectively, such that the device main body (4) and the heart rate detectors (3a and 3b) can be mounted on the user.

## Description

### BACKGROUND OF THE INVENTION

### FIELD OF THE INVENTION

The present invention relates to a biological information detecting device in which an electrode is attached onto a surface of a living body to detect a biological signal.

### BACKGROUND ART

One of such biological information detecting devices is a heart rate measuring device that detects an electrocardiographic signal, which is generated in accordance with the beating of the heart, to measure a heart rate of a living body (for example, refer to JP-A-6-245913).

The heart rate measuring device (corresponding to the "biological information detecting device" of the Claims) disclosed in JP-A-6-245913 is formed as a belt-equipped type and includes a chest belt that is attached onto the chest. The chest belt includes a transmitter having an electrode that is in contact with a surface of a living body and detects an electrocardiographic signal; and an elastic support band (corresponding to the "fixing means" of the Claims) that is connected to both ends of the transmitter and elastically supports the transmitter on the chest of a human body.

It is generally known that the detection accuracy of the biological information detecting device depends on the adhesion between the electrode of the biological information detecting device and a surface of a living body. In the heart beat detector disclosed in JP-A-6-245913, a predetermined fastening force is obtained by the elastic support band. Therefore, it is considered that, for example, during exercise, the release of the electrode of the transmitter from a surface of a living body is prevented, and the electrode of the biological information detecting device is brought into close contact with a surface of a living body.

However, a chest circumference of a user onto which the biological information detecting device is mounted varies between individuals. Therefore, it is preferable that the total length of the support band be appropriately adjustable according to a chest circumference of a user such that the electrode of the transmitter is reliably brought into close contact with a surface of a living body. Accordingly, for example, a configuration can be conceived in which length adjusting means for adjusting the effective length of the support band is provided on the support band that elastically supports the transmitter.

It is known that the length adjusting means has, for example, the following configuration. That is, a center shaft is provided in a frame-shaped adjusting member, one end of the support band is attached onto the center shaft, and the other end thereof is drawn out from the center shaft and is folded back, such that a folded-double portion of the support band is formed between the center shaft and the folded portion of the support band. In addition, by inserting the other end of the support band into the frame-shaped adjusting member and drawing the other end out therefrom, a single, unfolded portion is formed between the center shaft and the other end of the support band. The adjusting member moves along a longitudinal direction of the support band, and a ratio of the folded-double portion and the single, unfolded portion of the support band is changed so as to adjust the effective length of the support band. This length adjusting portion is a so-called buckle.

However, in the above-described related art, fine adjustment is required for obtaining a predetermined fastening force during the mounting of the biological information detecting device while adjusting the effective length of the support band. Particularly, when the ratio of the folded-double portion and the single, unfolded portion of the support band is changed, a spring constant of the entire support band is changed. Therefore, in order to obtain a predetermined fastening force during the mounting of the biological information detecting device while adjusting the effective length of the support band, it is required that the fine adjustment of the effective length of the support band be performed in response to the change in the spring constant caused by the adjustment of the effective length of the support band. As a result, in the related art, it is difficult to adjust the support band such that the electrode is brought into contact with a surface of a living body by a predetermined pressing force and stable detectability can be secured.

### SUMMARY OF THE INVENTION

It is an aspect of the present application to provide a biological information detecting device which is capable of adjusting the effective length of fixing means according to a physical size of a living body and bringing an electrode into contact with a surface of a living body due to a predetermined pressing force to secure stable detectability.

According to the present application, there is provided a biological information detecting device including: a device main body; a biological signal detector that is integrally provided with the device main body and includes an electrode in contact with a surface of a living body; and fixing means for mounting the device main body and the biological signal detector on the living body, the fixing means being formed of a band-shaped member extendable in a longitudinal direction thereof. In this biological information detecting device, the fixing means includes a plurality of adjusting portions that are able to adjust an effective length of the fixing means, the plurality of adjusting portions include a rough adjusting portion and a fine adjusting portion, the rough adjusting portion adjusts the effective length of the fixing means so as to mount the device main body and the biological signal detector on the living body, and the fine adjusting portion adjusts a pressing force of the electrode against the surface of the living body.

According to this configuration, the rough adjusting portion roughly adjusts the length of the fixing means such that the device main body and the biological signal detector can be mounted on the living body, and the fine adjusting portion finely adjusts the length of the fixing means such that the pressing force of the electrode against the surface of the living body is a predetermined pressing force. Accordingly, the effective length of the fixing means can be adjusted according to a physical size of a living body, and the electrode can be brought into contact with the surface of the living body by the predetermined pressing force to secure stable detectability. In addition, in the related art, it is necessary that various sizes (for example, S-size, M-size, or L-size) of fixing means be provided according to a size of a living body. On the other hand, according to this configuration, since the length of the fixing means can be roughly adjusted according to a size of a living body, it is not necessary that various sizes of fixing means be prepared.

In addition, the adjusting portion may include an overlapping portion, where a part of the fixing means overlaps each other, and may adjust the length of the overlapping portion to adjust the pressing force of the electrode against the surface of the living body.

According to this configuration, since the length of the overlapping portion can be adjusted to adjust the pressing force of the electrode against the surface of the living body, an appropriate pressing force can be easily obtained. In addition, for example, after a user wears the biological information detecting device, even when the fixing means is loosened during use and the pressing force of the electrode is decreased, the overlapping portion can be pulled to readjust the length of the overlapping portion, thereby easily obtaining an appropriate pressing force. Accordingly, further stable detectability can be secured.

In addition, the adjusting portion may function as the rough adjusting portion by adjusting the length of the overlapping portion before the device main body and the biological signal detector are mounted on the living body, and the adjusting portion may function as the fine adjusting portion by adjusting the length of the overlapping portion after the device main body and the biological signal detector are mounted on the living body.

According to this configuration, the adjusting portion functions as the rough adjusting portion before the device main body and the biological signal detector are mounted on the living body, and the adjusting portion functions as the fine adjusting portion after the device main body and the biological signal detector are mounted on the living body. Therefore, even when the fixing means is loosened after a user wears the device main body and the biological signal detector, the adjusting portion can function as the fine adjusting portion and be readjusted.

In addition, the adjusting portion may include a holding member that holds the fixing means in a state where a part of the fixing means overlaps each other.

According to this configuration, by providing the holding member, the adjusting portion that finely adjusts the pressing force of the electrode against a surface of a living body to be a predetermined pressing force can be formed at a low cost. In addition, the holding member holds the fixing means in a state where a part of the fixing means overlaps each other. Therefore, after the adjusting portion adjusts a pressing force of the electrode against the surface of the living body, the pressing force can be maintained. Accordingly, further stable detectability can be maintained.

In addition, according to the present application, there is provided a biological information detecting device including: a device main body; a biological signal detector that is integrally provided with the device main body and includes an electrode in contact with a surface of a living body; and fixing means for mounting the device main body and the biological signal detector on the living body, the fixing means being formed in a band shape. In the biological information detecting device, the fixing means includes an adjusting portion and a non-adjusting portion, the adjusting portion includes an overlapping portion, where a part of the fixing means overlaps each other, and adjusts the length of the overlapping portion so as to adjust an effective length of the fixing means, the non-adjusting portion is formed in a predetermined length, the adjusting portion is formed of a member non-extendable in a longitudinal direction of the fixing means, and the non-adjusting portion is formed of an elastic member extendable in the longitudinal direction.

According to this configuration, the fixing means includes the adjusting portion that adjusts the length of the overlapping portion to adjust the effective length of the fixing means. Therefore, the effective length of the fixing means can be adjusted according to a physical size of a living body. In addition, the adjusting portion is formed of a member non-extendable in a longitudinal direction of the fixing means. Therefore, even when the length of the overlapping portion is adjusted to change a ratio of the overlapping portion in the fixing means, a change in the spring constant of the fixing means can be prevented.

In addition, the fixing means includes the adjusting portion and the non-adjusting portion, the adjusting portion is formed of a member non-extendable in the longitudinal direction of the fixing means, and the non-adjusting portion is formed in a predetermined length and is formed of an elastic member extendable in the longitudinal direction. Therefore, irrespective of the ratio of the overlapping portion, the spring constant of the fixing means can be maintained to be constant in the fixing means. As a result, even when the effective length of the fixing means is adjusted, a predetermined fastening force can be easily obtained. Therefore, the electrode can be brought into contact with a surface of a living body by the predetermined pressing force irrespective of the effective length of the fixing means to secure stable detectability.

According to the application, a biological information detecting device can be provided which is capable of adjusting the effective length of fixing means according to a physical size of a living body and bringing an electrode into contact with a surface of a living body due to a predetermined pressing force to secure stable detectability.

### BRIEF DESCRIPTION OF THE DRAWINGS

Embodiments of the present invention will now be described by way of further example only and with reference to the accompanying drawings, in which:
Fig. 1 is a diagram illustrating a state where a biological information detecting device according to a first embodiment of the present invention is mounted on a user.
Fig. 2 is a front view illustrating a heart rate measuring device and a fixing band according to the first embodiment.
Fig. 3 is a rear view illustrating the heart rate measuring device.
Fig. 4 is a cross-sectional view taken along line A-A of Fig. 2 illustrating the fixing band.
Fig. 5 is a perspective view illustrating a holding member.
Fig. 6 is a front view illustrating a heart rate measuring device and a fixing band according to a second embodiment of the present invention.
Fig. 7 is a cross-sectional view taken along line B-B of Fig. 6 illustrating the fixing band.
Fig. 8 is a graph illustrating a relationship between an elongation per unit length of the fixing band according to the second embodiment and a chest circumference of a user.

### DETAILED DESCRIPTION OF THE INVENTION

Hereinafter, a first embodiment of the present invention will be described using the drawings.

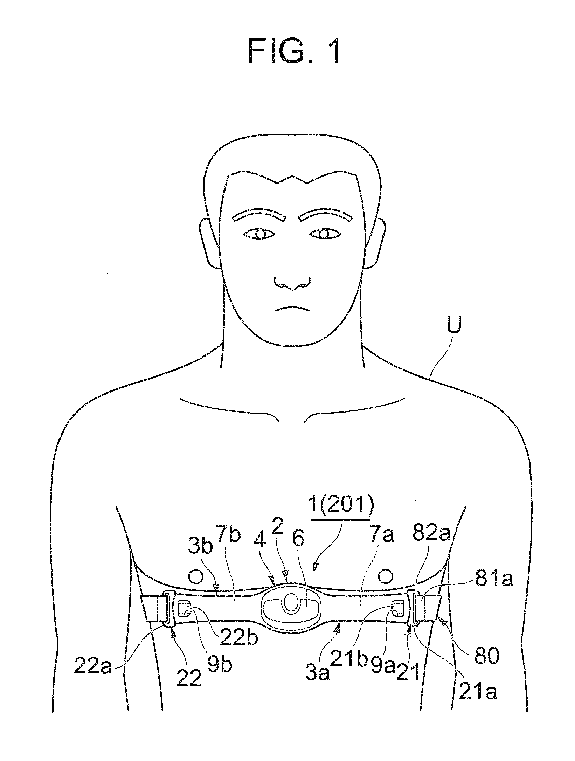

Fig. 1 is a diagram illustrating a state where a heart rate measuring device 1, which is a biological information detecting device according to the first embodiment, is mounted on a user U.

As illustrated in Fig. 1, the heart rate measuring device 1 is mounted on the chest, which is a body surface of the user U, and detects an electrocardiographic signal which is generated in accordance with the beating of the heart. Hereinafter, in a state being mounted on the user U, a side of the heart rate measuring device 1 in contact with the user U will be described as "rear side", and a side thereof which is opposite the rear side and faces outward will be described as "front side". In addition, in the following description, a direction following a circumferential direction of the chest of the user U will also be simply referred to as "circumferential direction of the chest".

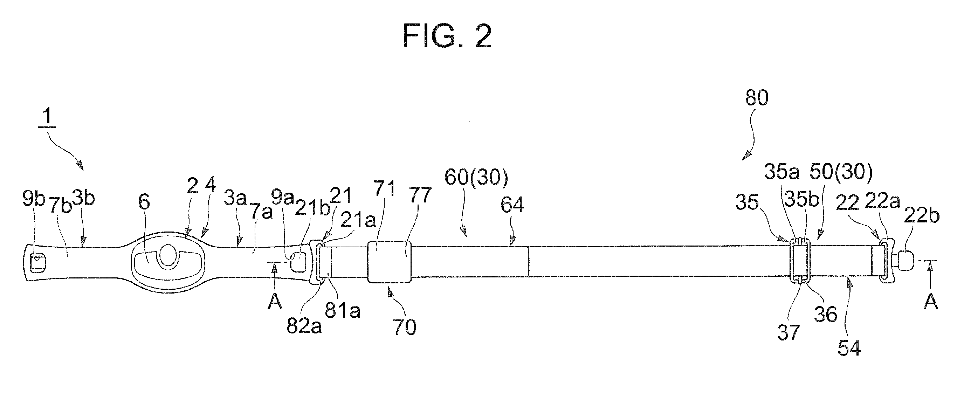

Fig. 2 is a front view illustrating the heart rate measuring device 1 and a fixing band 80 according to the first embodiment.

As illustrated in Fig. 2, the heart rate measuring device 1 includes a device main body 2; a pair of heart rate detectors (corresponding to "biological signal detector" of Claims) 3a and 3b that are integrally provided with the device main body 2 and include a pair of electrodes 7a and 7b in contact with a surface of a living body; a main body belt 4 that integrally covers the device main body 2 and the pair of heart rate detectors 3a and 3b; and the fixing band 80 (corresponding to the "fixing means" of the Claims) for mounting the device main body 2 and the pair of heart rate detectors 3a and 3b on the user U (refer to Fig. 1). In Fig. 2, only one end of the fixing band 80 is connected to the main body belt 4.

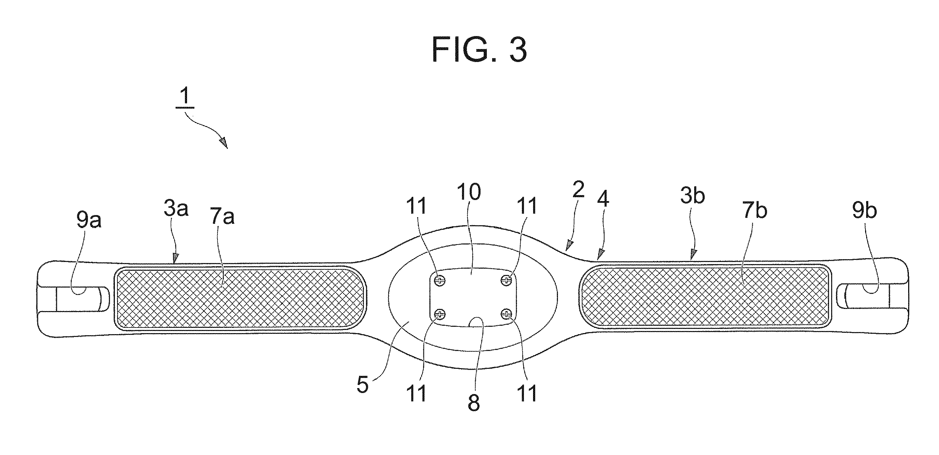

Fig. 3 is a rear view of the heart rate measuring device 1. In Fig. 3, the fixing band 80 (refer to Fig. 2) is not illustrated.

As illustrated in Fig. 3, the device main body 2 includes an elliptical case 5 that has a long axis along the circumferential direction of the chest. On the center of the rear side of the case 5, a battery housing 8 is formed, and a button-type battery (not illustrated) is housed in the battery housing 8. Further, in the battery housing 8 of the case 5, a battery lid 10 is fastened and fixed by screws 11 such that the battery housing 8 is sealed in a state where the button-type battery is housed therein.

As illustrated in Fig. 2, on the front side of the case 5, a cover glass 6 that covers the front side of the case 5 is provided. The cover glass 6 is formed such that the outermost surface is flush with the main body belt 4 and improves the appearance.

As illustrated in Fig. 3, the pair of heart rate detectors 3a and 3b are connected to ends of the device main body 2 on both sides in the circumferential direction of the chest, respectively, and are configured by the pair of electrodes 7a and 7b. The pair of electrodes 7a and 7b are formed of, for example, a band-shaped conductive elastomer. One end of each electrode in the circumferential direction of the chest is electrically connected to a circuit substrate (not illustrated) in the case 5. Examples of the conductive elastomer include a conductive silicone rubber into which carbon black is incorporated, a conductive rubber into which carbon black is incorporated, and a conductive polyurethane rubber into which carbon black is incorporated.

The main body belt 4 that integrally covers the device main body 2 and the pair of heart rate detectors 3a and 3b configured as above is formed of, for example, a urethane rubber in a band shape along the circumferential direction of the chest, and the total length thereof is approximately, for example, 260 mm. The main body belt 4 is formed so as to cover an edge of the device main body 2 on the rear side and edges of the pair of electrodes 7a and 7b. As illustrated in Fig. 2, fixing band attaching portions 9a and 9b are formed at both ends of the main body belt 4, respectively. The fixing band attaching portions 9a and 9b are formed of, for example, through-holes which penetrate the main body belt 4 in a thickness direction thereof.

### Fixing Band

The fixing band 80 has a longitudinal direction along the circumferential direction of the chest and is formed of an elastic belt-shaped member, such as a rubber material, extendable in the longitudinal direction.

The fixing band 80 includes, as adjusting portions 30 that adjust the effective length of the fixing band 80 (that is, the heart rate measuring device 1), a first adjusting portion 50 that includes an adjusting member 35; and a second adjusting portion 60 that is provided to be closer to a first side (left side in Fig. 2) of the longitudinal direction than the adjusting member 35 and includes a holding member 70.

The fixing band 80 adjusts the first adjusting portion 50 and the second adjusting portion 60 such that the effective length of the heart rate measuring device 1 is set to be slightly shorter than that of the chest circumference (refer to Fig. 1) of the user U to generate a predetermined fastening force in the heart rate measuring device 1. As a result, the fixing band 80 brings the pair of electrodes 7a and 7b into contact with the body surface of the user U with a predetermined pressing force.

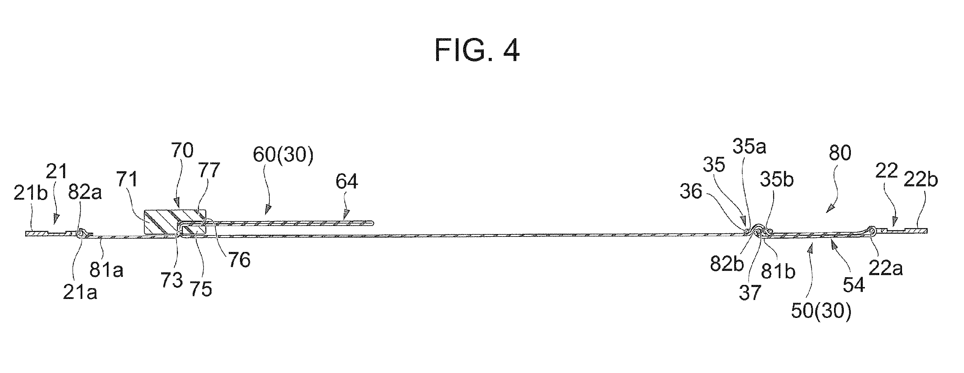

Fig. 4 is a cross-sectional view taken along line A-A of Fig. 2 illustrating the fixing band 80. In Fig. 4, the left side is the first side of the fixing band 80 in the longitudinal direction, and the right side is a second side of the fixing band 80 in the longitudinal direction. In addition, the upper side in Fig. 4 is the front side, and the lower side is the rear side.

As illustrated in Fig. 4, at an end 81a of the fixing band 80 on the first side and an end 81b thereof on the second side, ring portions 82a and 82b are formed such that terminals thereof are folded back, respectively.

The end 81a of the fixing band 80 on the first side is connected to a first engaging member 21 which can be engaged with the fixing band attaching portion 9a (refer to Fig. 2) of the main body belt 4.

The first engaging member 21 is a member, which is formed of, for example, a resin material, and includes a ring-shaped portion 21a and an engaging piece 21b. The ring-shaped portion 21a is inserted into the ring portion 82a of the fixing band 80 and is connected to the end 81a of the fixing band 80 on the first side. The engaging piece 21b is integrally formed with the ring-shaped portion 21a and is formed to be engaged with the fixing band attaching portion 9a of the main body belt 4.

The end 81b of the fixing band 80 on the second side is connected to the adjusting member 35.

The adjusting member 35 is formed in, for example, a rectangular frame shape (refer to Fig. 2) when seen in a front view. A center shaft 37 is provided inside a frame portion 36 along a width direction intersecting with the longitudinal direction.

The center shaft 37 is inserted into the ring portion 82b which is provided at the end 81b of the fixing band 80 on the second side.

On the first side and the second side of the longitudinal direction with the center shaft 37 interposed therebetween, a first window 35a and a second window 35b are partitioned by the center shaft 37 and the frame portion 36 of the adjusting member 35. The adjusting member 35 is configured to be slidable along the longitudinal direction when being slid on a surface of the fixing band 80 on the front side.

In order for the device main body 2 and the heart rate detectors 3a and 3b (all of which can be referred to Fig. 1) to be mountable on the chest (refer to Fig. 1) of the user U, before the device main body 2 and the heart rate detectors 3a and 3b are mounted on the chest, the first adjusting portion 50 functions as a rough adjusting portion which roughly adjusts the effective length of the fixing band 80, and after the device main body 2 and the heart rate detectors 3a and 3b are mounted on the chest, the first adjusting portion 50 functions as a fine adjusting portion which finely adjusts the effective length of the fixing band 80 to adjust a pressing force of the pair of electrodes 7a and 7b against the chest of the user U. The first adjusting portion 50 includes the above-described adjusting member 35; and an overlapping portion 54 which is provided to be closer to the second side (right side in Fig. 2) than the adjusting member 35 in the fixing band 80.

The overlapping portion 54 is formed between the center shaft 37 and the second engaging member 22 by causing, in a state where the end 81b of the fixing band 80 on the second side is connected to the center shaft 37 of the adjusting member 35, the end 81a of the fixing band 80 on the first side to pass through a ring-shaped portion 22a of a second engaging member 22 from the rear side to the front side and folding the fixing band 80 back. Further, the end 81a of the fixing band 80 on the first side is inserted from the rear side into the second window 35b, which is partitioned by the center shaft 37 and the adjusting member 35, crosses the front side of the center shaft 37 over the end 81b of the fixing band 80 on the second side, is drawn out from the rear side of the first window 35a, and is connected to the first engaging member 21.

In the first adjusting portion 50, the adjusting member 35 moves to the first side (that is, the side of the first engaging member 21) of the longitudinal direction to increase a ratio of the overlapping portion 54 which is a folded-double portion of the fixing band 80 and to decrease a ratio of a single, unfolded portion of the fixing band 80 other than the overlapping portion 54. As a result, the effective length of the fixing band 80 can be reduced. In addition, the adjusting member 35 moves to the second side (that is, the side of the second engaging member 22) of the longitudinal direction to decrease a ratio of the overlapping portion 54 which is the folded-double portion of the fixing band 80 and to increase a ratio of the single, unfolded portion of the fixing band 80 except for the overlapping portion 54. As a result, the effective length of the fixing band 80 can be increased. By roughly adjusting the effective length of the fixing band 80 as described above, the device main body 2 and the heart rate detectors 3a and 3b can be mounted on the chest of the user U.

Before the device main body 2 and the heart rate detectors 3a and 3b are mounted on the chest, the second adjusting portion 60 functions as a rough adjusting portion which roughly adjusts the effective length of the fixing band 80, and after the device main body 2 and the heart rate detectors 3a and 3b are mounted on the chest, the second adjusting portion 60 functions as a fine adjusting portion which finely adjusts the effective length of the fixing band 80 to adjust a pressing force of the pair of electrodes 7a and 7b against the chest of the user U. The second adjusting portion 60 includes the holding member 70; and an overlapping portion 64 which is provided to be closer to the second side (right side in Fig. 2) than the holding member 70 in the fixing band 80.

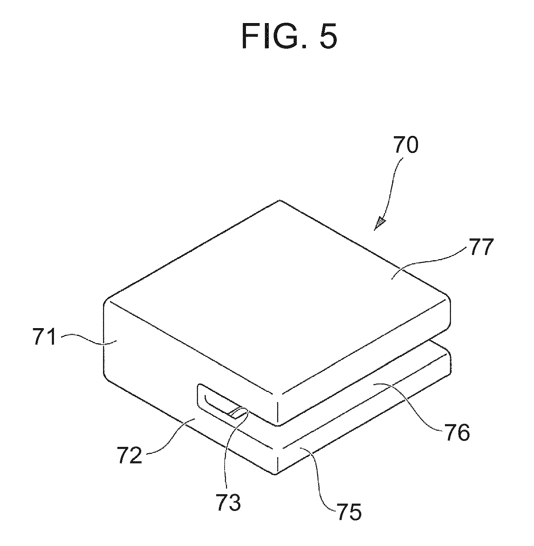

Fig. 5 is a perspective view illustrating the holding member 70.

As illustrated in Figs. 4 and 5, the holding member 70 is formed of, for example, a resin material and includes a holding member main body 71 that is formed in a block shape; a clamping piece 75 that is provided to be distant from the holding member main body 71 to the second side of the longitudinal direction of the fixing band 80 (refer to Fig.5); a connecting portion 72 that connects the holding member main body 71 and the clamping piece 75 on the outside of the fixing band 80 in the width direction (refer to Fig. 4); and a protrusion 77 that protrudes from the holding member main body 71 and is arranged opposite the clamping piece 75.

A space between the holding member main body 71 and the clamping piece 75 forms an insertion hole 73 into which the fixing band 80 is inserted. In addition, a space between the clamping piece 75 and the protrusion 77 forms a draw-out portion 76 for drawing out the fixing band 80, which is inserted into the insertion hole 73, to the second side of the longitudinal direction.

The overlapping portion 64 is formed by causing a part of the fixing band 80 between the first engaging member 21 and the adjusting member 35 to overlap each other. Thus, the overlapping portion 64 does not include either end 81a of the fixing band 80. The overlapping portion 64 is inserted into the insertion hole 73 and is drawn out to the second side (that is, the side of the second engaging member 22) of the longitudinal direction from the draw-out portion 76.

A part, corresponding to the insertion hole 73, of the overlapping portion 64 is clamped by the holding member main body 71 and the clamping piece 75 to be held with a predetermined holding force. In this case, when the device main body 2 and the heart rate detectors 3a and 3b are mounted on the chest of the user U to be used, the holding force of the holding member 70 is set to the extent that the fixing band 80 is not shifted between the holding member main body 71 and the clamping piece 75.

The second adjusting portion 60 allows the length of the overlapping portion 64 to be easily adjusted when being worn by the user to adjust a pressing force of the pair of electrodes 7a and 7b against the chest of the user U. Specifically, the overlapping portion 64, which is drawn out from the draw-out portion 76 of the holding member 70, is further drawn out to the second side of the longitudinal direction to increase a ratio of the overlapping portion 64 and to reduce the effective length of the fixing band 80. As a result, the pressing force of the pair of electrodes 7a and 7b against the chest of the user U can be increased. In addition, the fixing band 80 is drawn out from the insertion hole 73 of the holding member 70 to the first side and the second side of the longitudinal direction to decrease a ratio of the overlapping portion 64 and to increase the effective length of the fixing band 80. As a result, the pressing force of the pair of electrodes 7a and 7b against the chest of the user U can be reduced. By adjusting the length of the overlapping portion 64 as described above, the effective length of the fixing band 80 can be reduced or increased to finely adjust the effective length of the fixing band 80 and to finely adjust the pressing force of the pair of electrodes 7a and 7b against the chest of the user U.

### Operation

Next, hereinafter, the operation of the heart rate measuring device 1 according to the first embodiment will be described. It should be noted that the respective reference numerals in the following description can be referred to Figs. 1 to 5.

In such a configuration, the heart rate measuring device 1 is mounted on the chest of the user U (refer to Fig. 1) in the following procedure.

First, of the first engaging member 21 and the second engaging member 22 which are provided at both ends of the fixing band 80, for example, the engaging piece 21b of the first engaging member 21 which is provided on the first side of the longitudinal direction is engaged with the fixing band attaching portion 9a of the main body belt 4. As a result, the heart rate measuring device 1 is formed in a band shape by the end 81a of the fixing band 80 on the first side being connected to the main body belt 4.

Next, the user U slides the adjusting member 35 of the first adjusting portion 50 along the longitudinal direction to roughly adjust the effective length of the fixing band 80 such that the device main body 2 and the heart rate detectors 3a and 3b can be mounted on the chest of the user U. At this time, the first adjusting portion 50 functions as the rough adjusting portion which roughly adjusts the effective length of the fixing band 80.

Next, the user U engages the engaging piece 22b of the second engaging member 22, which is provided on the second side of the fixing band 80 in the longitudinal direction, with the fixing band attaching portion 9b of the main body belt 4. As a result, the heart rate measuring device 1 is attached on the chest of the user U in a ring shape.

Next, the user U adjusts the second adjusting portion 60 to finely adjust the effective length of the fixing band 80. As a result, a predetermined pressing force is applied to the pair of electrodes 7a and 7b, and the pair of electrodes 7a and 7b are brought into contact with the body surface of the user U by the predetermined pressing force. At this time, the second adjusting portion 60 functions as the fine adjusting portion which finely adjusts the effective length of the fixing band 80. Through the above-described processes, the mounting of the heart rate measuring device 1 on the user U is completed. The heart rate measuring device 1 detects an electrocardiographic signal, which is generated from the pair of electrodes 7a and 7b in accordance with the beating of the heart of the user, as biological information.

When the fixing band 80 is loosened after the mounting of the heart rate measuring device 1 on the user U, at least one of the first adjusting portion 50 and the second adjusting portion 60 is made to function as the fine adjusting portion to finely adjust the effective length of the fixing band 80. As a result, the pair of electrodes 7a and 7b are brought into contact with the chest by a predetermined pressing force. By providing the first adjusting portion 50 and the second adjusting portion 60 in the fixing band 80, an appropriate pressing force can be easily obtained.

### Effect of First Embodiment

According to the first embodiment, the first adjusting portion 50 roughly adjusts the length of the fixing band 80 such that the device main body 2 and the pair of electrodes 7a and 7b can be mounted on the body of the user U, and the second adjusting portion 60 finely adjusts the length of the fixing band such that the pressing force of the pair of electrodes 7a and 7b against the body surface of the user U is a predetermined pressing force. Accordingly, the effective length of the fixing band 80 can be adjusted according to a physical size of the user U, and the pair of electrodes 7a and 7b can be brought into contact with the body surface of the user U by a predetermined pressing force to secure stable detectability. In addition, in the related art, it is necessary that various sizes (for example, S-size, M-size, or L-size) of fixing bands 80 be provided according to a size of the user U. On the other hand, according to the first embodiment, since the length of the fixing band 80 can be roughly adjusted according to the size of the user U, it is not necessary that various sizes of fixing bands be prepared.

In addition, since the length of the overlapping portion 64 can be adjusted to adjust the pressing force of the pair of electrodes 7a and 7b against the body surface of the user U, an appropriate pressing force can be easily obtained. In addition, for example, after a user wears the heart rate measuring device 1, even when the fixing band 80 is loosened during use and the pressing force of the pair of electrodes 7a and 7b is decreased, the overlapping portion 64 can be pulled to readjust the length of the overlapping portion 64, thereby easily obtaining an appropriate pressing force. Accordingly, further stable detectability can be secured.

In addition, by providing the holding member 70, the second adjusting portion 60 that finely adjusts the pressing force of the pair of electrodes 7a and 7b against the body surface of the user U to be a predetermined pressing force can be formed at a low cost. In addition, the holding member 70 holds the fixing band 80 in a state where a part of the fixing band 80 overlaps each other. Therefore, after the second adjusting portion 60 adjusts a pressing force of the pair of electrodes 7a and 7b against the body surface of the user U, the pressing force can be maintained. Accordingly, further stable detectability can be maintained.

### Second Embodiment

Next, a heart rate measuring device 201 according to a second embodiment of the present invention will be described.

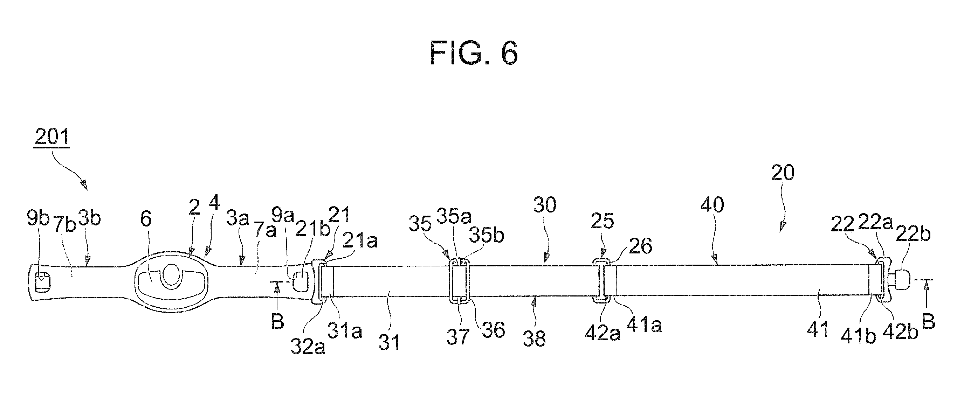

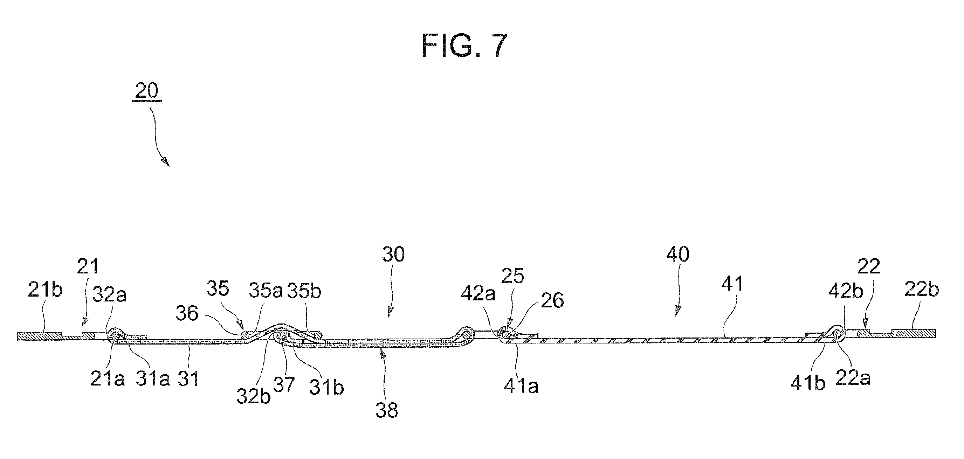

Fig. 6 is a front view illustrating the heart rate measuring device 201 and a fixing band 20 according to the second embodiment, and Fig. 7 is a cross-sectional view taken along line B-B of Fig. 6 illustrating the fixing band 20.

In the heart rate measuring device 1 according to the first embodiment, the fixing band 80 is formed of an elastic belt-shaped member, such as a rubber material, extendable in the longitudinal direction and includes the first adjusting portion 50 and the second adjusting portion 60 (refer to Fig. 2).

On the other hand, the heart rate measuring device 201 according to the second embodiment is different from that of the first embodiment, in that the heart rate measuring device 201 includes an adjusting portion 30 that is provided on the first side of the longitudinal direction and a non-adjusting portion 40 that is provided on the second side of the longitudinal direction. Hereinafter, only different configurations from that of the first embodiment will be described. In addition, it should be noted that the mounting state of the heart rate measuring device 201 according to the second embodiment on the user U is as shown in Fig. 1.

As illustrated in Figs. 6 and 7, the fixing band 20 is formed in a band shape in which the direction following the circumferential direction of the chest is a longitudinal direction thereof, and the fixing band 20 includes the adjusting portion 30 that is provided on the first side of the longitudinal direction and the non-adjusting portion 40 that is provided on the second side in the longitudinal direction. The adjusting portion 30 and the non-adjusting portion 40 are connected to each other through the ring-shaped connecting member 25.

### Adjusting Portion

The adjusting portion 30 includes an adjusting band 31 and an adjusting member 35.

The adjusting band 31 is formed of a member in a band shape, such as a cloth formed of a synthetic fiber or the like, non-extendable in the longitudinal direction. At an end 31a of the adjusting band 31 on the first side and an end 31b thereof on the second side, ring portions 32a and 32b are formed such that terminals thereof are folded back, respectively.

The end 31a of the adjusting band 31 on the first side is connected to the first engaging member 21 which can be engaged with the fixing band attaching portion 9a of the main body belt 4.

The first engaging member 21 is a member, which is formed of, for example, a resin material, and includes the ring-shaped portion 21a and the engaging piece 21b. The ring-shaped portion 21a is inserted into the ring portion 32a of the adjusting band 31 and is connected to the end 31a of the adjusting band 31 on the first side. The engaging piece 21b is integrally formed with the ring-shaped portion 21a and is formed to be engaged with the fixing band attaching portion 9a of the main body belt 4.

The end 31b of the adjusting band 31 on the second side is connected to the adjusting member 35.

On the second side of the longitudinal direction, the adjusting portion 30 includes an overlapping portion 38 where the adjusting band 31, which is a part of the fixing band 20, overlaps each other. The overlapping portion 38 is formed between the center shaft 37 and a connecting member 25 by causing, in a state where the end 31b of the adjusting band 31 on the second side is connected to the center shaft 37 of the adjusting member 35, the end 31a of the adjusting band 31 on the first side to pass through the inside of a frame of the connecting member 25 from the rear side to the front side and folding the adjusting band 31 back. Further, the end 31a of the adjusting band 31 on the first side is inserted from the rear side into the second window 35b, which is partitioned by the center shaft 37 and the adjusting member 35, crosses the front side of the center shaft 37 over the end 31b of the adjusting band 31 on the second side, is drawn out from the rear side of the first window 35a, and is connected to the first engaging member 21.

The adjusting member 35 is configured to be slidable on a space between the first engaging member 21 and the connecting member 25 along the longitudinal direction when being slid on a surface of the adjusting portion 30 on the front side. As a result, a ratio of the overlapping portion 38 in the adjusting portion 30 can be changed, and the length of the adjusting portion 30 can be changed. Specifically, the adjusting member 35 moves to the first side (that is, the side of the first engaging member 21) of the longitudinal direction to increase a ratio of the overlapping portion 38 which is a folded-double portion of the adjusting band 31 and to decrease a ratio of a single, unfolded portion of the adjusting band 31 other than for the overlapping portion 38. As a result, the total length of the adjusting portion 30 can be reduced. In addition, the adjusting member 35 moves to the second side (that is, the side of the connecting member 25) of the longitudinal direction to decrease a ratio of the overlapping portion 38 which is the folded-double portion of the adjusting band 31 and to increase a ratio of the single, unfolded portion of the adjusting band 31 other than for the overlapping portion 38. As a result, the total length of the adjusting portion 30 can be increased. By reducing or increasing the total length of the adjusting band 31, the effective length of the fixing band 20 can be adjusted.

In this embodiment, the adjusting portion 30 is formed so as to have a total length of, for example, approximately 260 mm, which is substantially the same as that of the main body belt 4, when the adjusting member 35 is positioned in an intermediate portion (hereinafter, referred to as "reference position") of the adjusting portion 30 in the longitudinal direction. Therefore, the total length of the adjusting portion 30 can be adjusted, for example, in a range from 195 mm to 390 mm.

### Non-Adjusting Portion

The non-adjusting portion 40 is formed of a band-shaped non-adjusting band 41, such as an elastic member of a rubber material or the like, extendable in the longitudinal direction in a predetermined length. The non-adjusting portion 40 according to this embodiment is formed so as to have a total length of, for example, 260 mm which is substantially the same as that of the main body belt 4. In this case, as described above, the total length of the adjusting portion 30 can be adjusted, for example, in a range from 195 mm to 390 mm. Accordingly, the effective length of the fixing band 20 including the adjusting portion 30 and the non-adjusting portion 40 can be adjusted in a range from 455 mm to 650 mm.

At an end 41a of the non-adjusting band 41 on a first side and an end 41b thereof on a second side, ring portions 42a and 42b are formed such that terminals thereof are folded back, respectively.

The end 41a of the non-adjusting band 41 on the first side is connected to the connecting member 25 by a frame portion 26 of the connecting member 25 being inserted into the ring portion 42a.

In addition, the end 41b of the non-adjusting band 41 on the second side is connected to the second engaging member 22 which can be engaged with the fixing band attaching portion 9b of the main body belt 4. The second engaging member 22 includes the ring-shaped portion 22a and the engaging piece 22b and is formed in the same shape as that of the first engaging member 21. The end 41b of the non-adjusting band 41 on the second side is connected to the second engaging member 22 by the ring-shaped portion 22a of the second engaging member 22 being inserted into the ring portion 42b.

The above-described heart rate measuring device 201 is formed in a ring shape so as to be mounted on the chest of the user U by the first engaging member 21 and the second engaging member 22 of the fixing band 20 being engaged with the fixing band attaching portions 9a and 9b of the main body belt 4, respectively (refer to Fig. 1). The length of the heart rate measuring device 201 according to this embodiment in the circumferential direction is, for example, approximately 780 mm when the adjusting member 35 of the adjusting portion 30 is positioned in the reference position.

In addition, as described above, the effective length of the fixing band 20 according to this embodiment can be adjusted in a range from 455 mm to 650 mm. Accordingly, the effective length of the heart rate measuring device 201 according to this embodiment in the circumferential direction of the chest (hereinafter, referred to as "the effective length of the heart rate measuring device 201") can be adjusted in a range from 715 mm to 910 mm.

The fixing band 20 according to this embodiment includes the adjusting portion 30 that is non-extendable in the longitudinal direction; and the non-adjusting portion 40 that is extendable in the longitudinal direction. Accordingly, by setting the effective length of the heart rate measuring device 201 to be slightly shorter than the chest circumference of the user U, the non-adjusting portion 40 can be elastically deformed and extend when the heart rate measuring device 201 is mounted on the chest of the user U. A predetermined fastening force is generated in the heart rate measuring device 201 by an elastic restoring force of the non-adjusting portion 40. Therefore, a predetermined pressing force is applied to the pair of electrodes 7a and 7b of the heart rate measuring device 201, and the pair of electrodes 7a and 7b are brought into contact with the body surface of the user U.

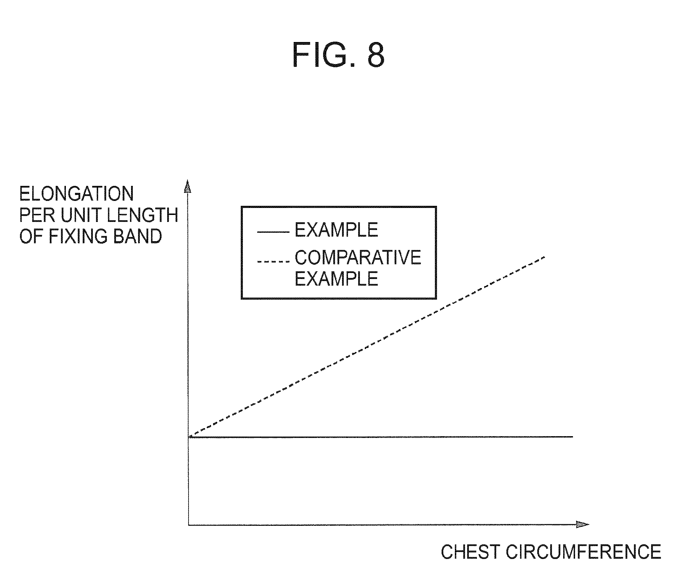

Fig. 8 is a graph illustrating a relationship between a chest circumference of the user U and an elongation per unit length of the fixing band 20 when the horizontal axis represents the chest circumference and the vertical axis represents the elongation. In Fig. 8, a broken line is a graph of a comparative example illustrating a chest circumference of the user U and an elongation per unit length of the fixing band 20 when a folded-double portion is provided in an extendable fixing band of the related art. It should be noted that the respective reference numerals in the following description can be referred to Figs. 1, 6, and 7.

In a heart rate measuring device according to the comparative example, a ratio of a folded-double portion having a high spring constant and a single, unfolded portion having a low spring constant is changed depending on the chest circumference of the user U. Specifically, as the chest circumference of the user U is increased, a ratio of the folded-double portion having a high fastening force in the fixing band is decreased, and a ratio of the single, unfolded portion having a low fastening force is increased. Therefore, the elongation of the fixing band per unit length tends to increase (refer to the broken line in Fig. 8), and it is difficult to adjust the effective length of the fixing band such that a predetermined fastening force can be secured.

On the other hand, the heart rate measuring device 201 according to the second embodiment includes the adjusting portion 30 that is formed of a member, such as a cloth formed of a synthetic fiber or the like, non-extendable in the longitudinal direction. Therefore, by adjusting the length of the overlapping portion 38, even when a ratio of the overlapping portion 38 in the fixing band 20 is changed, the length of the adjusting portion 30 is not changed. That is, the elongation of the adjusting portion 30 per unit length with respect to the chest circumference of the user U is always constant (refer to a solid line in Fig. 8).

As a result, by summing up the length which is constant with respect to the chest circumference of the user U, the length of the fixing band 20, in which a predetermined fastening force can be obtained according to the chest circumference of the user U, can be calculated. Therefore, the user U can easily adjust the effective length of the fixing band according to the chest circumference. Accordingly, irrespective of the effective length of the fixing band 20, the pair of electrodes 7a and 7b of the heart rate measuring device 201 are brought into contact with the body surface of the user U by a predetermined pressing force, and the stable detectability of the heart rate measuring device 201 can be secured.

### Operation

Next, hereinafter, the operation of the heart rate measuring device 201 according to this embodiment will be described.

In such a configuration, the heart rate measuring device 201 is mounted on the chest of the user U in the following procedure.

First, in the first engaging member 21 and the second engaging member 22 which are provided at both ends of the fixing band 20, for example, the engaging piece 21b of the first engaging member 21 which is provided on the first side of the longitudinal direction is engaged with the fixing band attaching portion 9a of the main body belt 4. As a result, the heart rate measuring device 201 is formed in a band shape by an end of the fixing band 20 on a first side being connected to the main body belt 4.

Next, the effective length of the fixing band 20 of the heart rate measuring device 201 is adjusted such that a predetermined fastening force can be obtained when the heart rate measuring device 201 is mounted on the chest of the user U. In this embodiment, for example, a graph (refer to Fig. 8), a table (not illustrated), and the like, which illustrate a relationship between the effective length of the fixing band 20 in which a predetermined fastening force can be obtained and the chest circumference of the user U, are included in an instruction manual and the like of the heart rate measuring device 201. Based on the graph, the table, and the like included in the instruction manual and the like, the user U adjusts the length of the adjusting portion 30 and adjusts the effective length of the fixing band 20. The effective length of the fixing band 20 is adjusted by sliding the adjusting member 35 on the surface of the adjusting portion 30 on the front side to be slid on the space between the first engaging member 21 and the connecting member 25 along the longitudinal direction. As a result, a ratio of the overlapping portion 38 in the adjusting portion 30 can be changed, and the length of the adjusting portion 30 can be changed, and thus the effective length of the fixing band 20 (that is, the effective length of the heart rate measuring device 201) can be adjusted.

Next, the band-shaped heart rate measuring device 201 in which the end of the fixing band 20 on the first side is connected to the main body belt 4 is wound around the chest of the user U. At this time, while arranging the device main body 2 on the chest center of the user U, the pair of electrodes 7a and 7b are brought into contact with the body surface of the user U.

The engaging piece 22b of the second engaging member 22, which is provided on the second side of the fixing band 20 in the longitudinal direction is engaged with the fixing band attaching portion 9b of the main body belt 4. As a result, the heart rate measuring device 201 is attached on the chest of the user U in a ring shape.

At this time, the effective length of the heart rate measuring device 201 is set to be slightly shorter than the chest circumference of the user U. Therefore, when the heart rate measuring device 201 is wound around the chest of the user U, the non-adjusting portion 40, which is formed of an elastic member, is elastically deformed and extends. A predetermined fastening force is generated in the heart rate measuring device 201 by an elastic restoring force of the non-adjusting portion 40, and a predetermined pressing force is applied to the pair of electrodes 7a and 7b to be brought into contact with the body surface of the user U.

Through the above-described processes, the mounting of the heart rate measuring device 201 on the user U is completed. The heart rate measuring device 201 detects an electrocardiographic signal, which is generated from the pair of electrodes 7a and 7b in accordance with the beating of the heart of the user U, as biological information.

### Effect of Second Embodiment

According to the second embodiment, the fixing means includes the adjusting portion 30 that adjusts the length of the overlapping portion 38 to adjust the effective length of the fixing band 20. Therefore, the effective length of the fixing band 20 can be adjusted according to a physical size of the user U. In addition, the adjusting portion 30 is formed of a member non-extendable in the longitudinal direction. Therefore, even when the length of the overlapping portion 38 is adjusted to change a ratio of the overlapping portion 38 in the fixing band 20, a change in the spring constant of the fixing band 20 can be prevented.

In addition, the fixing band 20 includes the adjusting portion 30 and the non-adjusting portion 40, the adjusting portion 30 is formed of a member non-extendable in the longitudinal direction of the fixing band 20, and the non-adjusting portion 40 is formed in a predetermined length and is formed of an elastic member extendable in the longitudinal direction. Therefore, the spring constant of the fixing band 20 can be maintained to be constant irrespective of the ratio of the overlapping portion 38 in the fixing band 20. As a result, even when the effective length of the fixing band 20 is adjusted, a predetermined fastening force can be easily obtained. Therefore, irrespective of the effective length of the fixing band 20, the pair of electrodes 7a and 7b of the heart rate measuring device 201 can be brought into contact with the body surface of the user U by the predetermined pressing force to secure the stable detectability of the heart rate measuring device 201.

The present invention is not limited to the above-described embodiments, and various modifications can be made for the above-described embodiments within a range not departing from the scope of the present invention.

The shape of the holding member 70 which constitutes the second adjusting portion 60 is not limited to that of the first embodiment and may be any shape as long as the holding member 70 holds the fixing band 80 in a state where the overlapping portion 64 is formed. Accordingly, the holding member 70 may be a clip which can clip the fixing band 80 in a state where a part of the fixing band 80 overlaps with each other. In addition, a configuration may be adopted in which a button or the like is provided on a part of the fixing band 80 such that the fixing band 80 can be held in a state where the overlapping portion 64 is formed.

In addition, in the first embodiment, a part, corresponding to the insertion hole 73, of the overlapping portion 64 of the fixing band 80 is clamped by the holding member main body 71 and the clamping piece 75 to be held with a predetermined holding force. On the other hand, a claw portion that can lock the overlapping portion 64 of the fixing band 80 may be provided on, for example, the draw-out portion 76 or the protrusion 77 of the holding member 70, the inside of the insertion hole 73, or the like.

In the first embodiment, the fixing band 80 includes, as adjusting portions 30 that adjust the effective length of the heart rate measuring device 1, one first adjusting portion 50 and one second adjusting portion 60. However, the numbers of the first adjusting portions 50 and the second adjusting portions 60 are not limited to one. Accordingly, the fixing band 80 may include, as the adjusting portion 30, plural first adjusting portions 50 and plural second adjusting portions 60.

In the first and second embodiments, the heart rate measuring device 201 that measures the heart rate of the user U is described as the biological information detecting device, in which the device main body 2 and the pair of heart rate detectors 3a and 3b are integrally provided and are attached onto the chest of the user U through the fixing band 20. However, the present invention is not limited to this configuration. In addition, the embodiments of the present invention in which the heart rate measuring device is used as an example of the biological information detecting device are described. However, the present invention may be applied to, for example, devices for measuring a blood pressure, a body temperature, a muscle potential, and the like.

The fixing band 20 according to the second embodiment includes one adjusting portion 30 and one non-adjusting portion 40. However, the numbers of the adjusting portions 30 and the non-adjusting portions 40 are not limited to one. For example, a configuration may be adopted in which a fixing band is divided into three fixing bands, one adjusting portion is provided on each of both sides of the respective fixing bands in the longitudinal direction, and one non-adjusting portion is arranged between the respective adjusting portions. In addition, for example, a configuration may be adopted in which a fixing band is divided into three fixing bands, one non-adjusting portion is provided on each of both sides of the respective fixing bands in the longitudinal direction, and one adjusting portion is arranged between the respective non-adjusting portions.

Further, within a range not departing from the scope of the present invention, the components in the above-described embodiments can be appropriately replaced with well-known components of the related art.

## Claims

1. A biological information detecting device (1) comprising:
a device main body (4);
a biological signal detector that is integrally provided with the device main body and includes an electrode (7a, 7b) in contact with a surface of a living body (U); and
fixing means (80) for mounting the device main body and the biological signal detector on the living body, the fixing means being formed of a band-shaped member extendable in a longitudinal direction thereof,
wherein the fixing means includes a plurality of adjusting portions (30) that are able to adjust an effective length of the fixing means,
the plurality of adjusting portions include a rough adjusting portion (50) and a fine adjusting portion (60),
the rough adjusting portion adjusts the effective length of the fixing means so as to mount the device main body and the biological signal detector on the living body, and
the fine adjusting portion adjusts a pressing force of the electrode against the surface of the living body.

2. The biological information detecting device according to claim 1,
wherein the adjusting portion includes an overlapping portion (64), where a part of the fixing means overlaps each other, and adjusts the length of the overlapping portion to adjust the pressing force of the electrode against the surface of the living body.

3. The biological information detecting device according to claim 2,
wherein the adjusting portion functions as the rough adjusting portion by adjusting the length of the overlapping portion before the device main body and the biological signal detector are mounted on the living body, and
the adjusting portion functions as the fine adjusting portion by adjusting the length of the overlapping portion after the device main body and the biological signal detector are mounted on the living body.

4. The biological information detecting device according to any one of claims 1 to 3,
wherein the adjusting portion includes a holding member (70) that holds the fixing means in a state where a part of the fixing means overlaps each other.

5. A biological information detecting device (1) comprising:
a device main body (4);
a biological signal detector that is integrally provided with the device main body and includes an electrode (7a, 7b) in contact with a surface of a living body (U); and
fixing means (80) for mounting the device main body and the biological signal detector on the living body, the fixing means being formed in a band shape,
wherein the fixing means includes an adjusting portion (30) and a non-adjusting portion (40),
the adjusting portion includes an overlapping portion (38), where a part of the fixing means overlaps each other, and adjusts the length of the overlapping portion so as to adjust an effective length of the fixing means,
the non-adjusting portion (40) is formed in a predetermined length,
the adjusting portion (30) is formed of a member non-extendable in a longitudinal direction of the fixing means, and
the non-adjusting portion (40) is formed of an elastic member extendable in the longitudinal direction.
